# EUROPEAN PATENT APPLICATION

(11) **EP 3 275 493 A1**
(43) Date of publication of application: **31.01.2018**
(21) Application number: 16305990.0
(22) Date of filing: 29.07.2016
(51) Int. Cl.: A61M 16/06

(54) **RESPIRATORY MASK WITH ANTI-ROTATION SYSTEM AND MISALIGNMENT INDICATORS**

(71) Applicant: Air Liquide Medical Systems, 92160 Antony (FR)
(72) Inventor: SANDONI, Giuseppe, 25124 Brescia (IT); ALBERICI, Luca, 25030 Roncadelle (BS) (IT); MASSERDOTTI, Fulvio, 25075 Brescia (IT)
(74) Representative: Pittis, Olivier

(57) **Abstract**

A respiratory mask comprising a main body (1) comprising a first connecting structure (2); and a flexible cushion (11) comprising a second connecting structure (12) cooperating with the first connecting structure for connecting and maintaining the cushion integral with the main body. The cushion comprises at least one lodging (13), and the main body comprises at least one protruding element (3) that is at least partially inserted into said lodging when the cushion is correctly coupled to the main body. The main body comprises at least a first marking (4) located on its peripheral surface (1'), and the cushion (11) comprises at least a second marking (14) located on its peripheral surface (11'), the first and second markings being aligned when the cushion is correctly coupled to the main body. The respiratory mask is useable for treatment of respiratory disorders affecting child or adult patients, such as obstructive sleep apnea.

## Description

The invention concerns a respiratory mask, in particular a nasal mask, for adult or for pediatric uses in the treatment of respiratory conditions or diseases affecting adults, children, toddlers, infants or newborns, comprising a cushion coupled to a main body, and an improved coupling system that prevents any wrong positioning of the cushion vis-a-vis the main body of the mask.

Respiratory masks, such as nasal masks, are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD)....

A respiratory mask assembly typically comprises a main body comprising a hollow shell, usually made of polymer, defining a breathing chamber that receives at least a part of the patient's nose, and further a soft face-contacting cushion that comes into contact with the patient's face and conforms to the various facial contours of the patient face, said cushion being coupled to the main body. This cushion is usually made of soft, resilient elastomeric material, such as soft silicone or similar material.

A respiratory mask additionally comprises a headgear for correctly maintaining and/or securing the mask on the head of a patient, and often a forehead support for improving the stability of the mask, once secured on the face of a patient.

The mask assembly is secured to the wearer's head by straps or similar devices that can be adjusted to pull the mask against the face with sufficient force to achieve a gas tight seal between the mask and the wearer's face. Indeed, patients have generally to wear the masks overnight.

Examples of such masks are disclosed by EP-A-462701, EP-A-874667, EP-A-1972357 or WO-A-00/57942.

However, in practice, some gas leaks and discomfort for patients have been noticed with a lot of masks.

Some investigations have shown that the gas leaks are often due to a wrong positioning of the cushion vis-a-vis the main body of the mask.

Indeed, for many patients, coupling the cushion to the main body of the mask, for example for a cushion is worn out and must be changed, is a difficult operation as patients have some difficulties to know whether the coupling is correctly done, i.e. if the cushion and mask body are well-aligned or not.

Hence, the problem to be solved is to provide an improved respiratory mask architecture, especially a nasal mask, that has a simplified architecture thereby allowing an easy coupling of the cushion to the main body of the mask by the patients and that, once coupled, provides to the patients an efficient indication of right or wrong positioning of the cushion to the main body, i.e. if they are well or misaligned, thereby improving the gas tightness (i.e. seal) as well as the comfort of use for the patients in preventing the escape of gas that may result from undesired positioning of the cushion with respect to the main body of the mask.

The solution of the present invention concerns a respiratory mask, in particular a nasal mask, especially for patients suffering from respiratory conditions or diseases, comprising:
- a main body comprising a first connecting structure, and
- a flexible cushion comprising a second connecting structure, said second connecting structure cooperating with the first connecting structure of the main body for connecting and maintaining the flexible cushion integral with the main body, characterized in that:
- the flexible cushion comprises at least one lodging, and the main body comprises at least one protruding element, said protruding element being at least partially inserted into said lodging when the flexible cushion is correctly coupled to the main body, and
- the main body comprises at least a first making located on its peripheral surface, and the flexible cushion comprises at least a second marking located on its peripheral surface, the first making being aligned with the second marking when the flexible cushion is correctly coupled to the main body.

The respiratory mask according to the present invention can further comprise one or more of the following additional features:
- the main body is made of a rigid material, such as a plastic material or the like.
- the flexible cushion is made of a resilient material, such as silicone or the like.
- the protruding element of the main body is entirely or almost entirely inserted into the lodging of the flexible cushion, when the flexible cushion is correctly coupled to the main body.
- the protruding element forms a protuberance on the inner surface of the main body.
- the protruding element and the lodging have complementary shapes.
- the protruding element and the lodging have complementary V-shapes or any other shape.
- the lodging is a cut or a notch arranged in the outer wall of the cushion.
- the first making and the second marking have complementary shapes.
- the first making and the second marking are segments of line, but can of course have other suitable shapes.
- the first connecting structure of the main body is traversed by a first gas passage, and the second connecting structure of the flexible cushion is traversed by a second gas passage, said first gas passage and second gas passage being in fluid communication, when the flexible cushion is coupled to the main body, so that the gas can travel through said first and second gas passages.
- the first connecting structure and the second connecting structure comprise several walls having cylindrical shapes.
- the first connecting structure comprises a first inner cylindrical wall and a first outer cylindrical wall (i.e. first annular coupling walls), the first inner cylindrical wall being spaced from the first outer cylindrical wall by a first groove, i.e. a first annular-shape groove.
- the second connecting structure comprises a second inner cylindrical wall and a second outer cylindrical wall (i.e. second annular coupling walls), the second inner cylindrical wall being spaced from the second outer cylindrical wall by a second groove, i.e. a second annular-shape groove.
- the first inner and outer cylindrical walls and the first (annular) groove cooperate with the second inner and outer cylindrical and the second (annular) groove for connecting and maintaining the flexible cushion integral with the main body.
- the first inner cylindrical wall and the first outer cylindrical wall of the first connecting structure are arranged face-to-face around the first gas passage.
- the second inner cylindrical wall and the second outer cylindrical wall of the second connecting structure are arranged face-to-face around the second gas passage.
- the first outer cylindrical wall of the first connecting structure of the main body of the mask is squeezed between the second inner and outer cylindrical walls of the second connecting structure of the flexible cushion for thereby maintaining the flexible cushion firmly coupled to the main body of the mask.
- the first inner and outer cylindrical walls are coaxially arranged.
- the second inner and outer cylindrical walls are coaxially arranged.
- the first inner and outer cylindrical walls, and the second inner and outer cylindrical walls are coaxially arranged, when the flexible cushion is correctly coupled to the main body.
- the protruding element, while inserted into the lodging of the cushion, prevents any rotation of the flexible cushion vis-à-vis the main body, when the flexible cushion is correctly coupled to the main body.
- the first making and the second marking, when mutually aligned, constitute visual indicators for a user that the flexible cushion is correctly coupled to the main body, i.e. well-aligned.
- the main body is traversed by a first tubular piece comprising the first inner cylindrical wall of the first connecting structure.
- the main body comprises two lateral arms.
- the main body comprises a holding arm projecting upwardly from said main body.
- the two lateral arms and the holding arm are integral with the main body.
- the main body, the holding arm and of the two lateral arms are molded in one piece.
- the main body, the holding arm and of the two lateral arms are made of a plastic (i.e. polymer) material, preferably a plastic material chosen among polycarbonate (PC), polypropylene (PP) or nylon.
- the holding arm and of the two lateral arms are slightly flexible.
- the two lateral arms and the holding arm each comprises a proximal end integral fixed to the main body, and a free distal end.
- the protruding element is molded in one piece with the main body.
- the protruding element has a peripheral profile that matches the inner profile of the opening of the lodging.
- a hollow curved connector is fixed to the tubular-shape portion of the main body and rotatable with respect to said main body.
- a tubular connecting piece is detachably fixed to the hollow curved connector, and rotatable with respect to said hollow curved connector.
- the upstream end of the tubular connecting piece is configured for connecting a gas line thereto. In other words, the tubular connecting piece is adapted for receiving a flexible gas conduct or hose that feeds a respiratory gas under pressure to the respiratory mask.
- the hollow curved connector is in fluid communication with the main body thereby allowing a gas to circulate from the hollow curved connector to the main body, or vice versa.
- the hollow curved connector has general "L" shape or similar.
- it further comprises a headgear comprising several straps connected to the holding arm and of the two lateral arms. The headgear is used for maintaining and securing the mask in a desired position on the head of the patient and on at least a part of the facial region, in particular on the nasal region, of the patient. Preferably, the straps of the headgear are fixed to the free distal ends of the holding arm and/or of the two lateral arms.
- the holding arm and of the two lateral arms comprise a strap-fixing system in the region of the free distal ends of the holding arm and of the two lateral arms, that comprises at least one slot and/or fixing hook for fixing straps of a headgear.
- the strap-fixing system carried by the free distal end of the holding arm comprises at least one slot, preferably two slots, such as open slots. For instance, the slots can have a width of about between 8 to 35 mm.
- the strap-fixing system carried by each free distal ends of the two lateral arms comprises a fixing hook.
- the straps are made of polymer material or fabric material, or both.
- the tubular portion of the mask body is traversed by a central passage for conveying the respiratory gas, said central passage comprising an inlet orifice and an outlet orifice having each a diameter of preferably between 1 and 2.5 cm. The inlet orifice and the outlet orifice can have same or different diameters.
- the holding arm and of the two lateral arms have an elongated shape.
- the holding arm and of the two lateral arms have for example a band or ribbon shape. Other shapes are of course also possible.
- the holding arm has a length of about 2 to 12 cm, preferably of about 2 to 8 cm.
- each lateral arms has a length of about 3 to 10 cm, preferably of about 3 to 6 cm.
- the main body, the holding arm and of the two lateral arms are made of a flexible polymeric material so as to be slightly bendable toward the patient's face under the forces applied by the headgear, when said headgear is adjusted to the morphology of the patient's head.
- the flexible cushion comprises an inner chamber, i.e. a respiratory chamber, in fluid communication with the second gas passage, and a large aperture in fluid communication with the inner chamber and adapted for receiving at least part of the patient's nose, when the patient wears the mask. The aperture of the flexible cushion is also called "nose aperture".
- the aperture comprises a peripheral border configured to ensure a gas tightness between the mask and the patient's face, in particular the nasal region.
- the flexible cushion comprises a peripheral border comprising one or several membranes arranged around along or part of the peripheral border, i.e. at the periphery of the aperture of the respiratory inner chamber. In other words, the membrane(s) form(s) a flexible skirt around the along or part of the periphery of the aperture of the respiratory chamber so as to ensure an efficient gas tightness while being in contact with the patient's face, when the latter wears the mask.
- the membrane(s) is (are) be molded in one piece with the cushion body including the peripheral border.
- the membrane and the cushion are integral and formed by a single piece made of a resilient soft or semi-soft material, i.e. of silicone or similar.
- at least a region of the holding arm and of the two lateral arms is configured or shaped so as to match, the external profile, i.e. the outer contour, of the flexible cushion.
- the holding arm and/or the two lateral arms are configured to have a slightly curved-shape.
- the mask is a nasal mask.
- the mask is a nasal mask and the cushion is configured and sized for coming into contact, when the mask is worn by the user or patient, with specific regions of the user or patient's face. Preferably, those regions comprise an intermediate nose region, an upper lip region and lateral nose regions, wherein:
   - the upper lip region is the area comprised between the nose and the upper lip;
   - the intermediate nose region is the area of the nose approximately located at the junction of bone and cartilage; and
   - the lateral nose regions are those located on each side of the nose.

The invention also concerns a respiratory assembly comprising a gas delivery device, such as a medical ventilator, and a respiratory mask according to the present invention, preferably the gas delivery device is connected to the respiratory mask by means of a gas line, such as a flexible hose. Typically, the gas line is connected to the tubular connecting piece fixed to a hollow curved connector, said hollow curved connector being itself fixed to the tubular portion of the main body of the mask.

An embodiment of a respiratory mask, especially a nasal mask, according to the present invention is shown in the enclosed Figures, among which:
- Figure 1 represents a rear view of the main body and a front view of the flexible cushion of a mask according to the present invention,
- Figure 2 is a lateral view of the flexible cushion coupled to the main body of Figure 1,
- Figure 3 and 4 show different embodiments of complementary markings located on the cushion and on the main body of the mask of Figure 1,
- Figure 5 and 6 show the details of the connecting structures for coupling the cushion to the mask body, and
- Figure 7 represents a view of an embodiment of a mask according to the present invention.

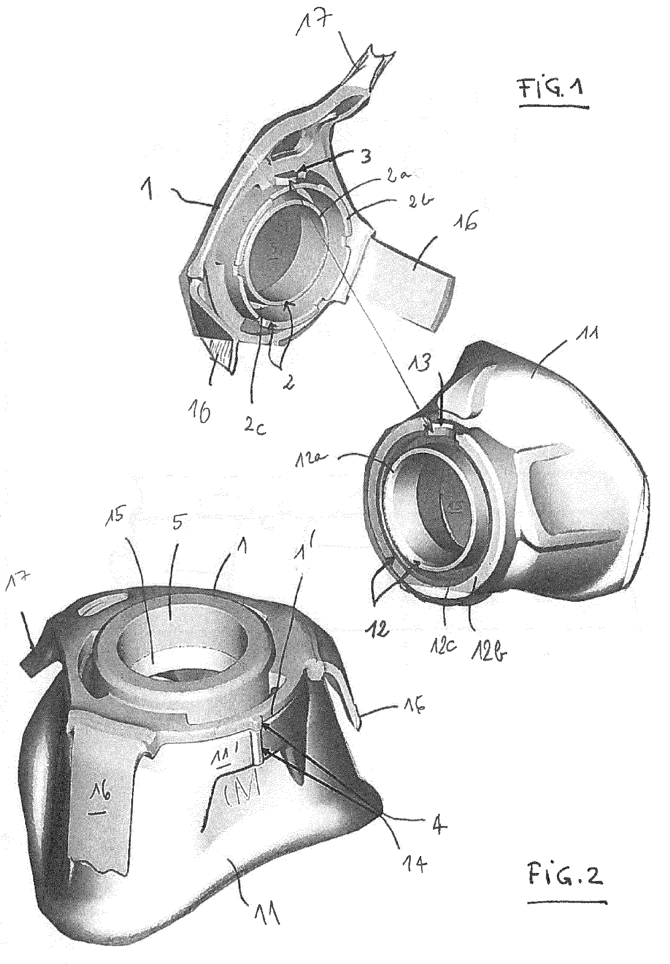

Figures 1-4 illustrate one embodiment of a nasal mask according to the present invention. Generally speaking, a respiratory mask according to the present invention comprises two main parts that can be assembled together or disassembled one from the other, namely a mask body or main body 1 and a flexible cushion 11, as shown in Figure 7.

The mask body 1 has a particular simple and light structure as it is formed of a rigid body 1 traversed by a first gas passage 5, i.e. a central passage, as visible in Figures 1, 2 and 7. The main body 1 of the mask according to the invention further carries two lateral arms 16 and a holding arm 17 that are integrally fixed to the external wall or surface of said main body 1.

As shown in Figure 7, the holding arm 17 projects upwardly, i.e. about vertically, from said main body 1, whereas the two lateral arms 16 project laterally from said main body 1 in opposite directions, i.e. one toward the right side of the mask and the other toward the left side of the mask as shown in Figure 1.

The holding arm 17 constitutes a frontal support that comes into contact with the patient's forehead. It's free end 17a generally carries a forehead pad or similar.

The two opposite lateral arms 16, also called right and left arms, constitute right and left cheek supports, when the mask is worn by a patient, that come into contact with the cheek regions of the patient.

Preferably, the main body 1, the two lateral arms 16 and the holding arm 17 are molded in one piece. Typically, they are made of a polymer material that is slightly flexible, preferably a plastic material, such as polypropylene (PP), polycarbonate (PC) and nylon, or similar. Nevertheless, they can be made from several pieces or sub-units that are subsequently assembled together and made integral by means of any available fixing technique, for instance they can be glued, thermo-welded or similarly affixed to one another. They can also be made of several and/or different materials.

Advantageously, the two lateral arms 16 and the holding arm 17 have elongated shapes, such as band or ribbon -like forms, and are slightly incurved, as shown in Figure 7, so as to match the contours of the cushion 11, i.e. configured or shaped so as to conform to the external profile of the flexible cushion 11.

The two lateral arms 16 and the holding arm 17 each comprise a free distal end 16a, 17a comprising a strap-fixing system 18, such as slots, hooks or similar, for fixing thereto the straps of a headgear (not shown) that is used for maintaining and securing the mask in a desired position on the head of the patient. Generally, the straps of headgears are made of polymer or fabric materials.

The mask of the present invention further comprises a flexible cushion 11 that is fixed to the rear side of the main body 1 as shown in Figure 1. Typically, the flexible cushion 11 is a tridimensional hollow flexible structure forming a respiratory inner chamber with a nose aperture adapted, i.e. conformed and sized, for receiving at least part of the patient's nose, when the patient wears the mask, so that the patient can breathe the respiratory gas contained in said inner chamber.

The cushion further comprises a second gas passage 15 for allowing a gas or gas mixture, such as under pressure air, to enter into the respiratory chamber of the cushion 11. Typically, the second gas passage 15 is located opposite to the nose aperture as shown in Figure 1. In order to provide efficient gas tightness (i.e. seal) and/or increased comfort for the patient, the nose aperture of the cushion 11 can be delimited by at least one flexible membrane that comes into contact with the patient's face and matches his/her facial morphology. The flexible membrane is arranged along all or part of the border of the nose aperture. Actually, the membrane(s) constitute(s) a kind of soft flexible skirt delimiting said the nose aperture of the cushion 11. Preferably, a unique or two superimposed membranes is/are used. The cushion 11 has a general shape matching the contours of the patient's face, especially in the nasal regions. When the mask is worn by the patient, i.e. when the cushion 11 receives at least a part of the patient's nose, when said patient introduces his/her nose into the internal volume of the respiratory inner chamber and breathes the gas contained therein, the cushion 11 and the membrane(s) arranged around the peripheral border of the nose aperture are in contact with the intermediate nose region (area at the junction of the bone and cartilage of the nose), the upper lip region (area between the nose and the upper lip), and the two opposite lateral regions of the nose of the patient (i.e. right and left flange regions of the nose). Of course, other embodiments and shapes are possible.

The cushion 11 is preferably made of a flexible and soft material, such as silicone or the like. Preferably, the membrane(s) and the cushion body are molded in one piece and made of the same flexible soft material.

The flexible cushion 11 is fixed to the main body 1 by means of a connecting system.

More precisely, the main body 1 comprises a first connecting structure 2 that is traversed by a first gas passage 5, and the flexible cushion 11 comprises a second connecting structure 12 that is traversed by a second gas passage 15.

The second connecting structure 12 cooperates with the first connecting structure 2 of the main body 1 for connecting and maintaining the flexible cushion 11 integral with the main body 1. The first gas passage 5 and the second gas passage 15 are in fluid communication, when the flexible cushion 11 is coupled to the main body 1.

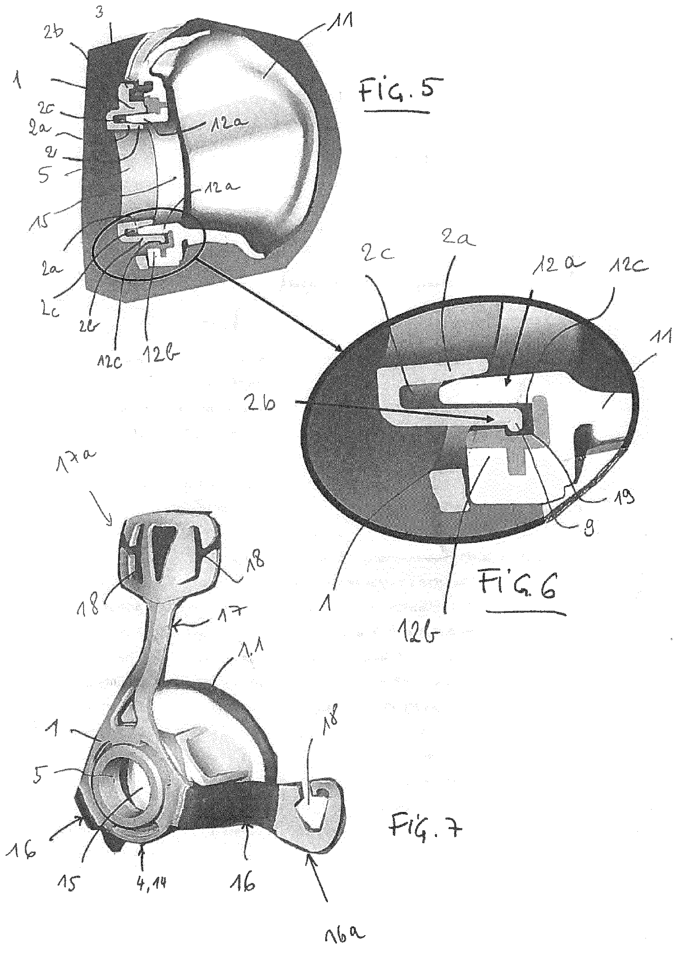

For obtaining a efficient coupling of the cushion 11 with the main body 1, the first connecting structure 2 and the second connecting structure 12 both comprise several walls 2a, 2b; 12a, 12b having cylindrical shapes, which cooperates together as shown in Figures 5 and 6.

More precisely, the first connecting structure 2 comprises a first inner cylindrical wall 2a and a first outer cylindrical wall 2b, the first inner cylindrical wall 2a being spaced from the first outer cylindrical wall 2b by a first groove 2c, i.e. a first annular groove, whereas the second connecting structure 12 comprises a second inner cylindrical wall 12a and a second outer cylindrical wall 12b, the second inner cylindrical wall 12a being spaced from the second outer cylindrical wall 12b by a second groove 12c, i.e. a second annular groove.

For connecting and maintaining the flexible cushion 11 fixed to and integral with the main body 1, the first inner and outer cylindrical walls 2a, 2b and the first (annular) groove 2c cooperate with the second inner and outer cylindrical walls 12a, 12b and the second annular groove 12c.

In other words, the outer wall 2b of the main body 1 is positioned in the second annular groove 12c of the cushion 11, and sandwiched and squeezed between the second inner and outer cylindrical walls 12a, 12b of the cushion 11 so as to hold the cushion 11 fixed to the main body 1 of the mask and, at the same time, to ensure a good gas tightness in-between.

For maintaining the cushion 11 firmly in position integral with the main body 1 of the mask, the free end of the outer wall 2b of the main body 1 can be shaped so as to present a kind of hook or bent finger 9 that cooperates with a cavity 19 arranged down in the groove 12c in the wall of the outer wall 2b as shown in Figure 6.

In the aim of providing a suitable alignment of the cushion 11 vis-a-vis the main body 1 and prevent any undesired rotation of the cushion 11 vis-a-vis the the main body 1 due to the fact that the cushion 11 is fixed to the main body 1 by cylindrical, i.e. tubular structures, according to the present invention, at least one lodging 13 is provided on the cushion 11 as shown in Figure 1, whereas at least one protruding element 3 is provided on the main body 1 which projects away from the inner surface of the main body 1, as shown in Figure 1, i.e. the protruding element 3 projects toward the cushion 11.

The lodging 13 cooperates with the protruding element 3 for preventing any undesired rotation of the cushion 11 vis-a-vis the mask body 1, thereby avoiding gas leaks and discomfort for the patient. Actually, the protruding element 3 is at least partially inserted into the lodging 13 of the main body 1, when the flexible cushion 11 is coupled or assembled to the main body 1 as shown in Figure 2.

In other words, the protruding element 3 has a suitable shape that is adapted to penetrate into the lodging 13 of the cushion 11, thereby acting as an abutment that prevents any undesired rotation of the flexible cushion 11 with respect to the main body 1, and further constituting, at the same time, a kind of foolproofing element that avoids unsuitable connections of the flexible cushion 11 to the main body 1.

Preferably, the protruding element 3 and the lodging 13 have complementary shapes. In other words, the protruding element 3 has a profile that matches the inner profile of the lodging 13. For example, the protruding element 3 and the lodging 13 have both V-shapes as shown in Figure 1. But, of course, any other shapes are useable as long as they cooperate so as to form an anti-rotation system that prevents any undesired rotation of the flexible cushion 11 vis-a-vis the main body 1.

Preferably, the protruding element 3 is molded in one piece with the rest of the main body 1.

Further, the lodging 13 is advantageously a cut, a notch or similar arranged in the outer wall 12b of the cushion 11 as shown in Figure 1.

Furthermore, the main body 1 comprises at least a first making 4 located on its peripheral surface 1', preferably on it is lower surface as shown in Figures 2 and 7, and the flexible cushion 11 comprises at least a second marking 14 located on its peripheral surface 11', preferably on it is lower surface as shown in Figures 2 and 7, the first making 4 being aligned with the second marking 14, when the flexible cushion 11 is correctly coupled to the main body 1.

Preferably, the first making 4 and the second marking 14 have complementary forms, for instance they are both segments of line as shown in Figure 2.

The first and the second markings 4,14, when mutually aligned, constitute visual indicators for the user, i.e. the patient, indicating him/her that the flexible cushion 11 is correctly coupled to the main body 1, thereby avoiding gas leaks and discomfort of use due to a misalignment of the cushion 11 vis-a-vis the main body 1.

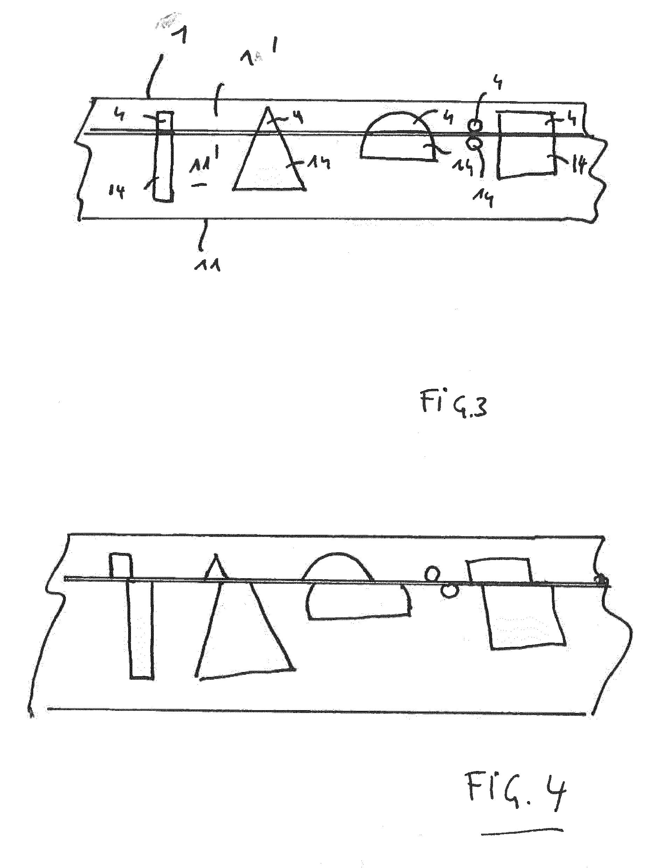

Figure 3 and 4 show different embodiments of complementary markings 4, 14 located on the cushion 11 and on the main body 1 of the mask of Figure 1 that are represented well-aligned (Fig. 3) and misaligned (Fig. 4).

Generally speaking, the respiratory mask of the present invention, such as a nasal mask, is light and comfortable to wear. Such a simple architecture leads to a reduction of the overall size and weight of the mask, thereby allowing efficient positioning and securing of the mask on the patient's face, as well as a good tightness (i.e. seal) and an improved comfort of use for the patient, especially thanks to the presence of the lodging 13 that cooperates with the protruding element 3 as explained above, and thanks to the first and the second markings 4, 14.

The respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

For carrying out such a method for treatment, the respiratory mask of the present invention can be used in a respiratory assembly comprising a gas delivery device and such a respiratory mask connected to the gas delivery device by means of a gas line, such as a flexible hose.

## Claims

1. Respiratory mask comprising :
- a main body (1) comprising a first connecting structure (2), and
- a flexible cushion (11) comprising a second connecting structure (12), said second connecting structure (12) cooperating with the first connecting structure (2) of the main body (1) for connecting and maintaining the flexible cushion (11) integral with the main body (1),
**characterized in that** :
- the flexible cushion (10) comprises at least one lodging (13), and the main body (1) comprises at least one protruding element (3), said protruding element (3) being at least partially inserted into said lodging (13) when the flexible cushion (11) is correctly coupled to the main body (1), and
- the main body (1) comprises at least a first making (4) located on its peripheral surface (1'), and the flexible cushion (11) comprises at least a second marking (14) located on its peripheral surface (11'), the first making (4) being aligned with the second marking (14) when the flexible cushion (11) is correctly coupled to the main body (1).

2. Mask according to the preceding Claim, **characterized in that** the protruding element (3) and the lodging (13) have complementary shapes.

3. Mask according to any one of the preceding Claims, **characterized in that** the protruding element (3) and the lodging (13) have complementary V-shapes.

4. Mask according to any one of the preceding Claims, **characterized in that** the first making (4) and the second marking (14) have complementary forms, preferably they are segments of line.

5. Mask according to any one of the preceding Claims, **characterized in that** the first connecting structure (2) of the main body (1) is traversed by a first gas passage (5), and the second connecting structure (12) of the flexible cushion (11) is traversed by a second gas passage (15), said first gas passage (3) and second gas passage (13) being in fluid communication when the flexible cushion (11) is coupled to the main body (1).

6. Mask according to any one of the preceding Claims, **characterized in that** the first connecting structure (2) and the second connecting structure (12) comprise several walls (2a, 2b; 12a, 12b) having cylindrical shapes.

7. Mask according to any one of the preceding Claims, **characterized in that**:
- the first connecting structure (2) comprises a first inner cylindrical wall (2a) and a first outer cylindrical wall (2b), the first inner cylindrical wall (2a) being spaced from the first outer cylindrical wall (2b) by a first groove (2c), and
- the second connecting structure (12) comprises a second inner cylindrical wall (12a) and a second outer cylindrical wall (12b), the second inner cylindrical wall (12a) being spaced from the second outer cylindrical wall (12b) by a second groove (12c).

8. Mask according to any one of the preceding Claims, **characterized in that** the first inner and outer cylindrical walls (2a, 2b) and the first groove (2c) cooperate with the second inner and outer cylindrical walls (12a, 12b) and the second groove (12c) for connecting and maintaining the flexible cushion (11) integral with the main body (1).

9. Mask according to any one of the preceding Claims, **characterized in that** the protruding element (3), while inserted into the lodging (13) of the cushion (11), prevents any rotation of the flexible cushion (11) vis-à-vis the main body (1), when the flexible cushion (11) is correctly coupled to the main body (1).

10. Mask according to any one of the preceding Claims, **characterized in that** the first making (4) and the second marking (14), when mutually aligned, constitute visual indicators for a user that the flexible cushion (11) is correctly coupled to the main body (1).

11. Mask according to any one of the preceding Claims, **characterized in that** the lodging (13) is a cut or a notch arranged in the outer wall (12b) of the cushion (11).

12. Mask according to any one of the preceding Claims, **characterized in that** the first making (4) is located on the lower surface (1') of the main body (1) and the second marking (14) is located on the lower surface (11') of the cushion (11).

13. Respiratory assembly comprising a gas delivery device and a respiratory mask according to any one of the preceding Claims, preferably the gas delivery device is connected to the respiratory mask by means of a gas line, such as a flexible hose.
